# EUROPEAN PATENT SPECIFICATION

(11) **EP 4 240 364 B1**
(45) Date of publication and mention of the grant of the patent: **31.12.2025**
(21) Application number: 21810296.0
(22) Date of filing: 08.11.2021
(51) Int. Cl.: A61K 31/5383, A61K 9/00, A61K 31/407, A61P 27/02, A61P 29/00, A61P 31/04

(54) **OPHTHALMIC COMPOSITION CONTAINING LEVOFLOXACIN AND KETOROLAC, METHOD FOR THE PREPARATION AND USE THEREOF**
OPHTHALMISCHE ZUSAMMENSETZUNG MIT LEVOFLOXACIN UND KETOROLAC, VERFAHREN ZUR HERSTELLUNG UND VERWENDUNG DAVON
COMPOSITION OPHTALMIQUE CONTENANT DE LA LÉVOFLOXACINE ET DU KÉTOROLAC, SON PROCÉDÉ DE PRÉPARATION ET SON UTILISATION

(30) Priority: 09.11.2020 IT 202000026690
(43) Date of publication of application: 13.09.2023
(73) Proprietor: NTC s.r.l., 20124 Milano, (IT); Rafarm UK Limited, London W2 6BD (GB)
(72) Inventor: MARCELLONI, Luciano, 20147 MILANO (MI) (IT); BERTOCCHI, Federico, 20146 MILANO (MI) (IT); RASSIA, Ioanna, 19002 PEANIA ATTIKIS (GR); CHALKIAS, George, 19002 PEANIA ATTIKIS (GR); CHATZELLIS, Konstantinos, 19002 PEANIA ATTIKIS (GR); FOSTIERI, Efrosini, 19002 PEANIA ATTIKIS (GR)
(74) Representative: Bianchetti & Minoja with Trevisan & Cuonzo IPS SRL
(86) International application number: PCT/EP2021/080870
(87) International publication number: WO 2022/096702

(56) References cited:
- WO-A1-01/08689
- WO-A2-2005/101982
- FILIPE H P ET AL: "Microfluidic in vitro Drug Release from Contact Lens Materials", vol. 93, 1 October 2015 (2015-10-01), pages n/a, XP009515894, ISSN: 1755-375X, Retrieved from the Internet <URL:https://onlinelibrary.wiley.com/doi/abs/10.1111/j.1755-3768.2015.0578> [retrieved on 20150923], DOI: 10.1111/J.1755-3768.2015.0578
- KRESKEN MICHAEL ET AL: "Effectiveness of Levofloxacin Eye Drops - A Microbiological Perspective", vol. 02, no. 01, 1 January 2009 (2009-01-01), pages 12, XP055818778, ISSN: 1756-1795, Retrieved from the Internet <URL:https://www.touchophthalmology.com/wp-content/uploads/sites/16/2015/07/kaspar.pdf> DOI: 10.17925/EOR.2009.02.01.12

## Description

### TECHNICAL FIELD OF THE INVENTION

The present invention relates to a stable ophthalmic composition comprising a therapeutically effective amount of antibiotic levofloxacin or a pharmaceutically acceptable salt thereof, as a first active ingredient, and a therapeutically effective amount of non-steroidal anti-inflammatory and analgesic ketorolac or a pharmaceutically acceptable salt thereof, as a second active ingredient, a process for the preparation thereof and the use thereof in the treatment of infectious and inflammatory states of the eye, particularly conjunctivitis.

### BACKGROUND OF THE INVENTION

Eye infections can be superficial (conjunctivitis) or deep (keratitis) and require the use of antibiotics, anti-inflammatories and sometimes analgesics.

Conjunctivitis is a well-known eye disease that involves redness, purulent secretions and swelling due to infection of the external tissues of the eye district. These manifestations determine a painful symptomatology of burning that in severe cases can last several days, even in the presence of pharmacological treatments. Conjunctivitis is associated with infections which can be either bacterial or viral, and the severity of this pathology ranges from mild to very serious up to an eye loss in untreated cases.

The routine treatment used for bacterial conjunctivitis comprises the administration of antibiotic eye drops to each eye up to 8 times a day, at least during the first days of treatment. In case of bacterial infections, a conjunctival swab is generally recommended to identify the bacterial species and strain in order to prescribe the most effective antibiotic eye drops against the relevant bacterial species.

If the infection is associated with conspicuous inflammatory phenomena, the therapy may include a steroidal anti-inflammatory compound to be administered with the antibiotic. In the most serious cases, the first-line therapy is often based on the administration of eye drops containing the antibiotic and a corticosteroid, separately or in association.

There are already several combination products on the market comprising a corticosteroid along with an anti-infective drug, such as TobraDex^{®} by Alcon containing tobramycin and dexamethasone and Blephamide^{®} by Allergan containing sulfacetamide sodium and prednisolone acetate. WO 01/08689 discloses aqueous ophthalmic compositions comprising ofloxacin and ketorolac for use in treating ocular infections.

However, the prior art has encountered substantial difficulties with such combination comprising an anti-infective with a corticosteroid due to the many side effects caused by the use of a corticosteroid, such as the prolongation of the course of the viral/fungal/bacterial infection or even its worsening due to the immune suppression corticosteroids cause.

There are several non-steroidal anti-inflammatory drugs used in the treatment of eye-inflammation, with a greater or lesser pain-relieving ability, but none except ketorolac possess a strong analgesic effect that acts centrally at the level of neural pathways of pain perception, which is particularly beneficial since the eye is a highly innervated tissue.

Ketorolac is a molecule belonging to the category of NSAIDs, non-steroidal anti-inflammatory drugs, and is generally used in salified form with tromethamine. Ketorolac tromethamine is a white or almost white crystalline powder with a molecular weight of 376.4 g/mol. Its solubility profile shows a pH dependent trend, significantly increasing at pH value > 6.0.

In common clinical practice, treatment with Ketorolac eye drops is prescribed to counteract the pain resulting from superficial eye injuries. The dosage of Ketorolac tromethamine eye drops currently on the market (Acular^{®} by Allergan) generally involves treating each eye with 3 to 4 drops of 0.5% w/v solution for 3-4 weeks starting 24 hours before the cataract surgery.

Levofloxacin is a fluoroquinolone antibiotic. It has a molecular weight of 361.4 g/mol and is poorly soluble in water, but sufficiently lipophilic to penetrate the eyes. Levofloxacin has a broad spectrum of activity and a fairly long duration of action. Recent studies showed that it remains in the eye at levels above the minimum inhibitory concentration (MIC) for more than six hours.

The dosage of Levofloxacin eye drops currently on the market (Oftaquix^{®} by Santen) generally involves the administration of a 0.5% solution in each eye in an amount of 8 drops in the first 2 days and 4 drops in the remaining 3 days, for a total of 28 drops.

Still, a storage-stable ophthalmic solution comprising an antibiotic with a non-steroidal anti-inflammatory drug (NSAID) for the simultaneous treatment of inflammation and infections of the eye, in particular before and after operation on the eye, is not yet available on the market due to the fact that such combinations are not satisfactory in terms of compatibility of their active ingredients and storage stability.

Thus, there still exists a need to provide a storage-stable aqueous ophthalmic composition comprising an antibiotic with a non-steroidal anti-inflammatory drug (NSAID) for the simultaneous treatment of inflammation and infections of the eye, in particular before and after operation on the eye, wherein the ingredients do not adversely affect one another in terms of their effectiveness and also in terms of their stability, or even undergo chemical reactions with one another, which could lead to undesired by-products.

### SUMMARY OF THE INVENTION

It is, therefore, an object of the present invention to provide a storage-stable aqueous pharmaceutical composition for ophthalmic administration containing levofloxacin or pharmaceutically acceptable salt thereof, as a first active ingredient, and ketorolac or a pharmaceutically acceptable salt thereof, as a second active ingredient, which overcomes the deficiencies of the prior art.

It is another object of the present invention to provide a storage-stable ophthalmic solution containing levofloxacin or a pharmaceutically acceptable salt thereof, as a first active ingredient, and ketorolac or a pharmaceutically acceptable salt thereof, as a second active ingredient, which is safe and effective with sufficient shelf-life and good pharmacotechnical properties.

Moreover, it is another object of the present invention to provide a suitable process for the preparation of a storage-stable aqueous pharmaceutical composition for ophthalmic administration containing levofloxacin or pharmaceutically acceptable salt thereof, as a first active ingredient, and ketorolac or a pharmaceutically acceptable salt thereof, as a second active ingredient, which is effective and reproducible.

In accordance with the above objects of the present invention, a stable ophthalmic composition in the form of an aqueous solution is provided comprising a therapeutically effective amount of antibiotic levofloxacin or a pharmaceutically acceptable salt thereof, as a first active ingredient, and a therapeutically effective amount of non-steroidal anti-inflammatory and analgesic ketorolac or a pharmaceutically acceptable salt thereof, as a second active ingredient, wherein said first active ingredient is from 0.4 to 0.9% w/v and said second active ingredient is from 0.4 to 0.9% w/v, wherein the weight/volume percentages are expressed as g/100mL units, wherein said composition has a pH value ranging from 7.7 to 8.5.

According to another embodiment of the present invention, a process for the preparation of a stable ophthalmic composition in the form of an aqueous solution comprising a therapeutically effective amount of antibiotic levofloxacin or a pharmaceutically acceptable salt thereof, as a first active ingredient, and a therapeutically effective amount of non-steroidal anti-inflammatory and analgesic ketorolac or a pharmaceutically acceptable salt thereof, as a second active ingredient, wherein said first active ingredient is from 0.4 to 0.9% w/v and said second active ingredient is from 0.4 to 0.9% w/v, wherein the weight/volume percentages are expressed as g/100mL units, is provided, wherein said process comprises the following steps:
(a) dissolving in water for injection at least one of the excipients selected from tonicity agents, chelating agents and/or preservatives;
(b) adding the total amount of ketorolac or salt thereof, to the resulting solution of step (a) and stirring for appropriate time until completely dissolved and adjusting the pH to a value between 8.5 to 9.5 with suitable hydrochloric acid or sodium hydroxide solution;
(c) adding the total amount of Levofloxacin or salt thereof to the solution of step (b) and stirring until completely dissolved;
(d) adjusting the pH of the final solution to a pH value between 7.5 to 8.5, preferably from 7.7 to 8.5, more preferably from 7.9 to 8.3 and most preferably 8.0, with suitable hydrochloric acid or sodium hydroxide solution.

Further, according to another embodiment of the present invention, a process for the preparation of a stable ophthalmic composition in the form of an aqueous solution comprising a therapeutically effective amount of antibiotic levofloxacin or a pharmaceutically acceptable salt thereof, as a first active ingredient, and a therapeutically effective amount of non-steroidal anti-inflammatory and analgesic ketorolac or a pharmaceutically acceptable salt thereof, as a second active ingredient, wherein said first active ingredient is from 0.4 to 0.9% w/v and said second active ingredient is from 0.4 to 0.9% w/v, wherein the weight/volume percentages are expressed as g/100mL units, is provided, wherein said process comprises the following steps:
(a) dissolving in water for injection at least one of the excipients selected from tonicity agents, chelating agents and/or preservatives;
(b) adding the total amount of levofloxacin or salt thereof, to the resulting solution of step (a) and stirring for appropriate time until completely dissolved and adjusting the pH to a value between 8.5 to 9.5 with suitable hydrochloric acid or sodium hydroxide solution;
(c) adding the total amount of ketorolac or salt thereof to the solution of step (b) and stirring until completely dissolved;
(d) adjusting the pH of the final solution to a pH value between 7.5 to 8.5, preferably from 7.7 to 8.5, more preferably from 7.9 to 8.3 and most preferably 8.0, with suitable hydrochloric acid or sodium hydroxide solution.

Further preferred embodiments of the present invention are defined in dependent claims 2 to 8 and 11 to 15.

Other advantages of the present invention will become apparent to those skilled in the art in view of the following detailed description.

### DETAILED DESCRIPTION OF THE INVENTION

The invention is defined by the claims. Any subject-matter falling outside the scope of the claims is provided for information purposes only. In particular, subject-matter relating to derivatives of levofloxacin and/or ketorolac is not according to the claimed invention.

For the purposes of the present invention, an aqueous pharmaceutical composition for ophthalmic administration comprising levofloxacin or pharmaceutically acceptable salt or derivative thereof, as a first active ingredient, and ketorolac or a pharmaceutically acceptable salt or derivative thereof, as a second active ingredient is considered to be "stable" if both said ingredients precipitate less or more slowly than they do on their own and/or in known pharmaceutical compositions during storage.

An excipient is considered to be "incompatible" with both said active ingredients or salts thereof if it promotes the degradation of said active ingredients, that is to say, if said active ingredients precipitate more or faster in the presence of said excipient when compared with the precipitation of said active ingredients on their own. The terms "incompatibility", "compatible" and "compatibility" are defined accordingly.

The aim of the present invention was the development of eye drops formulations containing a combination of levofloxacin and ketorolac in one unit dosage form in order to achieve strong therapeutic effects and to ensure patient compliance and convenience in the treatment of eye infection and/or inflammation. Under this scope, several different ophthalmic solutions were prepared and studied for their stability. In these studies, the formation of a solid precipitate was observed during product storage, across most of the tested concentration ranges of active ingredients and even using additives like solubilizers/surfactants.

It has been surprisingly found that the object of the present invention is achieved by adjusting the concentrations of the active ingredients and the pH value of the mixture at various steps during the manufacturing process and in the finished product, and by controlling the sequence of active ingredient addition, in order to prevent the formation of a precipitate and to improve the physicochemical stability of both active ingredients. Overcoming this technological hurdle enabled the preparation of a storage-stable composition containing effective amounts of levofloxacin and ketorolac.

Accordingly, in a first aspect the present disclosure provides a stable ophthalmic composition in the form of an aqueous solution comprising from about 0.4 to about 0.9% w/v levofloxacin and from about 0.4 to about 0.9% w/v ketorolac tromethamine expressed as weight/volume units (g/100 mL). In the ophthalmic composition of the present invention, levofloxacin can be used at different solvates or degrees of hydration, preferably as the hemihydrate.

In a preferred embodiment, the composition comprises about 0.5% w/v levofloxacin and about 0.5% w/v ketorolac tromethamine. In addition to levofloxacin and ketorolac, the composition of the invention may comprise excipients suitable for eye drops formulations and preferably tonicity agents, chelating agents, solubilizers/surfactants, pH buffers or adjusting agents and preservatives. In a preferred embodiment the composition contains, independently from one another: sodium chloride as tonicity agent; disodium EDTA as chelating agent; benzalkonium chloride as preservative; sodium hydroxide or hydrochloric acid to adjust the pH value.

To achieve stability of the product, the pH value of the composition is preferably adjusted in the range from about 7.5 to about 8.5. It was found that outside this pH range, a precipitate may form during product storage regardless of the addition of surfactants/solubilizers. The composition stability without the addition of surfactans and/or solubilizers was further increased when the pH was set in the range from about 7.7 to about 8.5 and especially around pH 8.0.

In the first basic studies on the present invention it was found that in the process of formulating an ophthalmic solution containing levofloxacin and ketorolac tromethamine unexpected stability problems arise when the pH of the composition was below 7.5, wherein a precipitate was observed, revealing that the two APIs are incompatible at a lower pH value. Best results in terms of absence of precipitate were obtained increasing the pH to 7.7 or more, up to pH 8.5, with particularly good results around pH 8.0.

Also, it was noticed that throughout addition of the drug components the pH of the solution dropped to approximately 5.3 prior to the addition of levofloxacin. According to the present invention, it was surprisingly found that by adding a step of intermediate pH adjustment in the middle of the manufacturing process to a higher pH value and preferably at 8.5-9.5 the stability of the product was significantly improved. Moreover, the sequence of ketorolac and levofloxacin addition at steps (b) and (c), in comparison to the reverse sequence seemed to have added robustness to the overall stability of the final product.

The ophthalmic composition of the present invention is used to prepare eye drops. To this purpose, pre-sterilized containers appropriate for ophthalmic use are filled with the eye drop solution optionally sealing the containers with dropper and screw cap in aseptic conditions. The containers can be single-dose or multi-dose, preferably plastic bottles, preferably opaque bottles, preferably made of polyethylene and equipped with LDPE droppers and polyethylene screw caps. The bottle can contain up to 10 mL, preferably between 2.5 and 10 mL and more preferably between 4 and 6 mL.

In a further aspect, the invention concerns the ophthalmic composition according to claims 1-8 for use in the therapeutic or preventive treatment of infectious and/or inflammatory states of the eye, in particular conjunctivitis optionally associated with infections of bacterial or viral origin, before or after eye surgery and particularly cataract surgery.

The following examples illustrate preferred embodiments in accordance with the present invention without limiting the scope of the invention
EXAMPLES (compositions in the examples comprising a pH beyond the claimed pH ranges and a concentration of levofloxacin and/or ketorolac in the ophthalmic composition beyond the claimed concentration ranges are reference compositions)

### Example 1 - Compatibility studies of the APIs Levofloxacin and Ketorolac tromethamine

**TABLE 1: Composition containing 0.5% Levofloxacin and 0.5% Ketorolac tromethamine in water**

| **Ingredient** | **Concentration (mg/mL)** | **Function** |
|---|---|---|
| Levofloxacin hemihydrate | 5.12 (5.00 on the anhydrous basis) | Active ingredient, anti-infective |
| Ketorolac tromethamine | 5.00 | Active ingredient, NSAID |
| Sodium hydroxide or Hydrochloric acid | q.s. to according to Table 2 | pH adjusting agent |
| Water for injection | q.s. 1 mL | Vehicle |

Unless otherwise indicated, all steps in this procedure were carried out at room temperature.

The preparation of Example 1 of the present invention was prepared according to the following process: Water for injection was added in the compounding vessel and the total amount of ketorolac tromethamine was added under stirring and when complete dissolution was achieved, levofloxacin hemihydrate was added and water for injection to the fill volume as required. The final pH value of the solution was measured with a calibrated pH-meter and, if necessary, it was adjusted to pH value according to Table 2 by using hydrochloric acid or sodium hydroxide solution. The results are presented below (Table 2).

**TABLE 2: Appearance monitoring of preparations of Example 1.**

| **Final pH** | **Results at 30 days** |
|---|---|
| 6.0 | Precipitated |
| 6.5 | Precipitated |
| 7.0 | Precipitated |
| 7.5 | Clear |
| 8.0 | Clear |

The presence of precipitate showed that the two active ingredients appear to be compatible with each other provided that the solution pH is 7.5 or higher.

Various compositions comprising other excipients as well, such as preservatives, chelating agents and tonicity agents were manufactured to investigate their impact on the formation of the precipitate.

### Example 2 - Compositions 1 and 2 containing 0.5% Levofloxacin and 0.5% Ketorolac tromethamine

**TABLE 3: Composition 1 and Composition 2 of the present invention.**

| | **Composition 1** | **Composition 2** | |
|---|---|---|---|
| **Ingredient** | **Concentration (mg/mL)** | **Concentration (mg/mL)** | **Function** |
| Levofloxacin hemihydrate | 5.12 (5.00 on the anhydrous basis) | 5.12 (5.00 on the anhydrous basis) | Active ingredient, anti-infective |
| Ketorolac tromethamine | 5.00 | 5.00 | Active ingredient, NSAID |
| Sodium chloride | q.s. 280-420 mOsm/kg osmolality | q.s. 280-420 mOsm/kg osmolality | Tonicity agent |
| Disodium EDTA | 1.00 | 1.00 | Chelating agent |
| Benzalkonium chloride | 0.10 | 0.10 | Preservative |
| Sodium hydroxide or Hydrochloric acid | q.s. **pH 6.5** | q.s. **pH 7.5** | pH adjusting agent |
| Water for injection | q.s. 1 mL | q.s. 1 mL | Vehicle |

Unless otherwise indicated, all steps in this procedure were carried out at room temperature.

Composition 1 and Composition 2 of the present invention were prepared according to the following manufacturing process: Water for injection about 90% of the total amount was added in the compounding vessel and the total amount of the excipients sodium chloride, disodium edetate and benzalkonium chloride were added under stirring until complete dissolution. Subsequently, ketorolac tromethamine was added under stirring until complete dissolution. Then, levofloxacin hemihydrate was added and the remaining amount of water for injection to the fill volume as required. The final pH of the solution was measured and, if required, it was adjusted to pH value approximately 6.5 for composition 1 and pH 7.5 for composition 2 by using hydrochloric acid or sodium hydroxide solution.

Upon monitoring, composition 2 at pH value 7.5 was clear throughout the observation period of 30 days, whereas the eye drop composition 1 at pH value 6.5 was observed to exhibit a precipitate soon after preparation. In the effort to reverse this outcome, the inventors of the present invention found that the precipitate was effectively dissolved and the solution became clear when the final pH value was gradually adjusted at or above pH 7.5 with the addition of a suitable electrolyte.

These results reveal that although the marketed formulations of levofloxacin have a pH approximately 6.0-6.8 and the formulations of ketorolac have a pH approximately 6.8-7.4, the combination of the two active ingredients would only present stability in a pH value not lower than 7.5. Studies with even higher pH values showed that the ideal final pH of the product should be around 8.0 for improved long-term stability. In particular it was found that the composition was increasingly stabilized at pH 7.7 and above, up to 8.5, with best results in the range 7.9-8.3 and particularly at pH 8.0.

### Example 3 - Compositions 3, 3a and 3b containing 0.5% Levofloxacin and 0.5% Ketorolac tromethamine (Composition 3 = Reference example)

**TABLE 4: Compositions 3, 3a and 3b of the present invention containing 0.5% Levofloxacin and 0.5% Ketorolac tromethamine.**

| | **Composition 3** | **Composition 3a** | **Composition 3b** | |
|---|---|---|---|---|
| **Ingredient** | **Concentration (mg/mL)** | **Concentration (mg/mL)** | **Concentration (mg/mL)** | **Function** |
| Levofloxacin hemihydrate | 5.12 (5.00 on the anhydrous basis) | 5.12 (5.00 on the anhydrous basis) | 5.12 (5.00 on the anhydrous basis) | Active ingredient, anti-infective |
| Ketorolac tromethamine | 5.00 | 5.00 | 5.00 | Active ingredient, NSAID |
| Sodium chloride | q.s. 280-420 mOsm/kg osmolality | q.s. 280-420 mOsm/kg osmolality | q.s. 280-420 mOsm/kg osmolality | Tonicity agent |
| Disodium EDTA | 1.00 | 1.00 | 1.00 | Chelating agent |
| Benzalkonium chloride | 0.10 | 0.10 | 0.10 | Preservative |
| Sodium hydroxide | q.s. **pH 8.0** | q.s. **pH 7.5** | q.s. **pH 7.7** | pH adjusting agent |
| Hydrochloric acid | | | | |
| Water for injection | q.s. 1 mL | q.s. 1 mL | q.s. 1 mL | Vehicle |

Unless otherwise indicated, all steps in this procedure were carried out at room temperature.

Compositions 3, 3a and 3b of Example 3 of the present invention were prepared according to the following manufacturing process: Water for injection about 90% of the total amount was added in the compounding vessel and the total amount of the excipients sodium chloride, disodium edetate and benzalkonium chloride were added under stirring until complete dissolution. Subsequently, ketorolac tromethamine was added under stirring until complete dissolution and a sample of the solution was obtained to measure the pH value using a calibrated pH-meter. With the addition of the appropriate amount of electrolyte the pH value of the main solution was adjusted to pH values 9.0, 10.0 and 11.0 according to Table 5. Then, levofloxacin hemihydrate was added and the remaining amount of water for injection to the fill volume as required. The final pH of the solution was measured and, if required, it was adjusted to pH value approximately 8.0 for composition 3, pH value 7.5 for composition 3a and pH value 7.7 for composition 3b by using hydrochloric acid or sodium hydroxide solution. The final solution was then passed through a sterilizing filter and filled into plastic bottles for ophthalmic use.

Compositions 1, 2, 3, 3a and 3b of the present invention highlight that a pH value of at least 7.5, preferably 7.7 and more preferably around 8.0 is a pre-requisite for the two APIs to be compatible with each other. During the manufacturing process of the eye drop solution, it was observed that several formulation ingredients including ketorolac tromethamine created a mild acidic environment before the addition of levofloxacin, thus leading the two APIs to become mixed in a physical stability-unfavourable pH environment. For that reason, the introduction of an intermediate pH adjustment step was explored. Several different pH values were tested within an adequately alkaline range to assure that incorporation of levofloxacin in the solution would take place while solution pH would consistently remain above pH value 7.5.

**TABLE 5. Appearance monitoring of compositions 3, 3a and 3b manufactured with high values of intermediate pH.**

| **Intermediate pH value during manufacture** | **Final pH 7.5 Composition 3a** | **Final pH 7.7 Composition 3b** | **Final pH 8.0 Composition 3** |
|---|---|---|---|
| **5.3 (no adjustment)** | **Precipitated after 450 days** | **Clear solution*** | **Clear solution*** |
| 9.0 | Clear solution* | Clear solution* | Clear solution* |
| 10.0 | Precipitated after 162 days | Precipitated after 202 days | Clear solution* |
| 11.0 | Precipitated after 710 days | Precipitated after 658 days | Precipitated after 308 days |

| | | | |
|---|---|---|---|
| Asterisk * indicates a result as monitored approximately 770 days after preparation. | | | |

The results showed that intermediate pH value at or above 10.0 affected negatively the stability of Compositions 3, 3a and 3b regardless the final pH value (see Table 5). Namely, when during manufacture the intermediate pH value was adjusted to 10.0 or 11.0 prior to the addition of levofloxacin the composition presented a precipitate after a few months. Thus, we further investigated the impact of intermediate pH adjustment at pH values between 8.5 and 9.5 on the stability of the product.

Therefore, compositions 3c, 3d and 3e were manufactured with intermediate pH values at 8.5, 9.0 and 9.5, respectively, according to the manufacturing process of Composition 3 and bottles of said compositions were exposed to long-term stability studies (25±2°C/ 60±5% relative humidity). The stability results over 12 months showed that when the intermediate pH value of the solution was adjusted to 8.5-9.5 before the addition of the second active ingredient, the formulation is stable and all physicochemical attributes comply with acceptance criteria (see Table 6).

**TABLE 6: Stability results of Compositions 3c, 3d and 3e at long-term conditions (25±2°C, 60±5% relative humidity)**

| **Formulation** | | **Composition 3c** | **Composition 3d** | **Composition 3e** |
|---|---|---|---|---|
| Intermediate pH | | 8.5 | 9.0 | 9.5 |
| Appearance | | Complies | Complies | Complies |
| Levofloxacin Assay (anhydrous basis) (%) | | 100.9 | 101.0 | 100.2 |
| Ketorolac assay (%) | | 99.5 | 100.6 | 99.8 |
| Total related substances of Levofloxacin (%) | | BQL | BQL | BQL |
| Total related substances of Ketorolac (%) | | BQL | BQL | BQL |
| Benzalkonium chloride assay (%) | | 92.9 | 93.4 | 92.8 |
| Final pH | | 8.0 | 8.0 | 8.0 |
| Particulate matter | > 10 µm/mL | 21 | 26 | 24 |
| | > 25 µm/mL | 1 | 1 | 1 |
| | > 50 µm/mL | 0 | 0 | 0 |

| | | | | |
|---|---|---|---|---|
| BQL states for below quantification limit Complies means clear solution, essentially free from visible particles | | | | |

The particulate matter was measured according to USP-NF 42-37 <789> *Particulate matter in ophthalmic solutions* wherein it defines the acceptable limits for particulate matter as: 50 for > 10 µm/mL, 5 for > 25 µm/mL and 2 for > 50 µm/mL.

### Example 4 - Composition 4 containing 0.5% Levofloxacin and 0.5% Ketorolac tromethamine and Polysorbate 20 as solubilizer.

**TABLE 7: Composition 4 of the present invention.**

| | **Composition 4** | |
|---|---|---|
| **Ingredient** | **Concentration (mg/mL)** | **Function** |
| Levofloxacin hemihydrate | 5.12 (5.00 on the anhydrous basis) | Active ingredient, anti-infective |
| Ketorolac tromethamine | 5.00 | Active ingredient, NSAID |
| Sodium chloride | q.s. 280-420 mOsm/kg osmolality | Tonicity agent |
| Disodium edetate | 1.00 | Chelating agent |
| Polysorbate 20, 10% w/v | 0.5 | Solubilizer |
| Benzalkonium chloride | 0.10 | Preservative |
| Sodium hydroxide or Hydrochloric acid | q.s. pH 8.0 | pH adjusting agent |
| Water for injection | q.s. 1 mL | Vehicle |

Unless otherwise indicated, all steps in this procedure were carried out at room temperature.

Composition 4 of Example 4 of the present invention was prepared according to the following manufacturing process: Water for injection about 90% of the total amount was added in the compounding vessel and the total amount of the excipients sodium chloride, disodium edetate and benzalkonium chloride were added under stirring until complete dissolution. Subsequently, ketorolac tromethamine was added under stirring until complete dissolution and the pH value at this stage was adjusted to approximately 9.0. The appropriate amount of Polysorbate 20 solution along with the remaining amount of water for injection and levofloxacin hemihydrate were added to the solution. The pH value of the final product was adjusted to 8.0 with the addition of either hydrochloric acid or sodium hydroxide. The final solution was then passed through a sterilizing filter and filled into plastic bottles for ophthalmic use.

Composition 4 of example 4 with various solubilizers/surfactants other than Polysorbate 20 have been tested and the results showed that the main factor that ensures stability to the combination product of levofloxacin and ketorolac is the final pH value.

The results are presented below in Table 8. In fact, at final pH value 7.5 or above and in particular in the pH range from about 7.7 to about 8.5 and especially around pH 8.0 the composition with no solubilizer and/or surfactant presented better stability, wherein all solutions containing a solubilizer and/or surfactant presented a precipitate after a few months. The results in Table 8 further support that the final pH value of the solution plays a crucial role to the stability of the product since a high pH value (such as pH value 8.0 or above) may even stabilize formulations containing solubilizers and/or surfactants which are unstable at a lower pH value.

**TABLE 8: Appearance monitoring of Composition 4 of the present invention with various solubilizers.**

| **Solubilizer used in Composition 4** | **Final pH 7.5** | **Final pH 8.0** |
|---|---|---|
| None | Clear solution* | Clear solution* |
| Polysorbate 20 | Precipitated after 114 days | Clear solution* |
| Octoxynol-40 | Precipitated after 114 days | Precipitated after 624 days |
| Poloxamer 407 | Precipitated after 50 days | Clear solution* |
| Polyoxyl 40 stearate | Precipitated after 39 days | Clear solution* |
| Tyloxapol | Precipitated after 78 days | Clear solution* |
| Polyoxyl-35 castor oil | Precipitated after 106 days | Clear solution* |

| | | |
|---|---|---|
| Asterisk * indicates a result as monitored at least 750 days after preparation | | |

### Example 5 - Composition 5 containing 0.5% Levofloxacin and 0.5% Ketorolac tromethamine at final pH 8.5

**TABLE 9: Composition 5 of the present invention.**

| | **Composition 5** | |
|---|---|---|
| **Ingredient** | **Concentration (mg/mL)** | **Function** |
| Levofloxacin hemihydrate | 5.12 (5.00 on the anhydrous basis) | Active ingredient, anti-infective |
| Ketorolac tromethamine | 5.00 | Active ingredient, NSAID |
| Sodium chloride | q.s. 280-420 mOsm/kg osmolality | Tonicity agent |
| Disodium EDTA | 1.00 | Chelating agent |
| Benzalkonium chloride | 0.10 | Preservative |
| Sodium hydroxide or Hydrochloric acid | q.s. **pH 8.5** | pH adjusting agent |
| Water for injection | q.s. 1 mL | Vehicle |

Composition 5 of Example 5 of the present invention was prepared according to the manufacturing process of Composition 3 and the intermediate pH value was adjusted to approximately 9.0 and the pH of the final product was adjusted to 8.5 with the addition of either hydrochloric acid or sodium hydroxide.

The bottles of Composition 5 of Example 5 were exposed to long-term (25±2°C, 60±5% relative humidity) stability studies. The stability results of Composition 5 over 3 months showed that said composition of levofloxacin and ketorolac tromethamine at final pH value of 8.5 is also stable (see Table 10) indicating that a higher pH value can be applied to said combination. However, a pH higher than 8.5 is not typically applied in ophthalmic solutions, since adverse effects to the eye application may appear (e.g. burning sensation, itching, etc.) due to the high difference with the physiological pH of the tear fluid which is 7.4.

**TABLE 10: Stability results of Composition 5 at long-term conditions (25±2°C, 60±5% relative humidity)**

| **Time interval** | | **1 month** | **2 months** | **3 months** |
|---|---|---|---|---|
| Appearance | | Complies | Complies | Complies |
| Levofloxacin Assay (anhydrous basis) (%) | | 100.7 | 102.6 | 101.5 |
| Ketorolac assay (%) | | 99.2 | 99.3 | 100.8 |
| Total related substances of Levofloxacin (%) | | <0.05 | 0.05 | 0.05 |
| Total related substances of Ketorolac (%) | | <0.05 | <0.05 | <0.05 |
| Benzalkonium chloride assay (%) | | 92.8 | 92.0 | 92.3 |
| Final pH | | 8.5 | 8.5 | 8.5 |
| Particulate matter | > 10 µm/mL | 14 | 16 | 16 |
| | > 25 µm/mL | 0 | 1 | 1 |
| | > 50 µm/mL | 0 | 0 | 0 |

| | | | | |
|---|---|---|---|---|
| Complies means clear solution, essentially free from visible particles | | | | |

The particulate matter was measured according to USP-NF 42-37 <789> *Particulate matter in ophthalmic solutions* wherein it defines the acceptable limits for particulate matter as: 50 for > 10 µm/mL, 5 for > 25 µm/mL and 2 for > 50 µm/mL.

### Example 6 -Compositions 3, 6, 7 and 8 containing various concentrations of Levofloxacin hemihydrate and Ketorolac tromethamine.

**TABLE 11: Compositions 3, 6, 7 and 8 of the present invention.**

| | **Composition 3** | **Composition 6** | **Composition 7** | **Composition 8** |
|---|---|---|---|---|
| **Ingredient** | **Concentration (mg/mL)** | | | |
| Levofloxacin hemihydrate | 5.12 (5.00 on the anhydrous basis) | 9.05 (8.75 on the anhydrous basis) | 10.33 (10.00 on the anhydrous basis) | 15.51 (15.00 on the anhydrous basis) |
| Ketorolac tromethamine | 5.00 | 8.75 | 10.00 | 15.00 |
| Sodium chloride | q.s. 280-420 mOsm/kg osmolality | q.s. 280-420 mOsm/kg osmolality | q.s. 280-420 mOsm/kg osmolality | q.s. 280-420 mOsm/kg osmolality |
| Disodium EDTA | 1.00 | 1.00 | 1.00 | 1.00 |
| Benzalkonium chloride | 0.10 | 0.10 | 0.10 | 0.10 |
| Sodium hydroxide or Hydrochloric acid | q.s. pH 8.0 | q.s. pH 8.0 | q.s. pH 8.0 | q.s. pH 8.0 |
| Water for injection | q.s. 1 mL | q.s. 1 mL | q.s. 1 mL | q.s. 1 mL |

Studies with higher concentrations of the active ingredients were conducted in order to investigate further the stability of the product. Compositions 6, 7 and 8 of Example 6 of the present invention were prepared according to the same manufacturing process of Composition 3 and the intermediate pH value was adjusted to approximately 9.0 and the pH of the final product was adjusted to 8.0 with the addition of either hydrochloric acid or sodium hydroxide.

The bottles of Composition 3, 6, 7 and 8 were exposed to long-term (25±2°C, 60±5% relative humidity) stability studies. The stability results of Compositions 3, 6, 7 and 8 over 1 month showed that high concentrations of the APIs lead to an unstable product (see Table 12), as it is determined from the increase in the number of particulate matter.

**TABLE 12: Stability results of Compositions 3, 6, 7 and 8 at long-term conditions (25±2°C, 60±5% relative humidity)**

| **Composition** | | **Composition 3** | **Composition 6** | **Composition 7** | **Composition 8** |
|---|---|---|---|---|---|
| Intermediate pH | | 9.0 | 9.0 | 9.0 | 9.0 |
| Appearance | | Complies | Complies | Visible particles | Visible particles |
| Levofloxacin Assay (anhydrous basis) (%) | | 101.0 | 101.8 | 103.5 | 101.4 |
| Ketorolac assay (%) | | 100.6 | 105.8 | 103.4 | 101.4 |
| Total related substances of Levofloxacin (%) | | BQL | <0.05 | BQL | <0.05 |
| Total related substances of Ketorolac (%) | | BQL | <0.05 | <0.05 | ND |
| Benzalkonium chloride assay (%) | | 93.4 | 100.4 | NP | 99.4 |
| Final pH | | 8.0 | 8.0 | 8.0 | 8.1 |
| Particulate matter | > 10 µm/mL | 26 | 21 | 81 | 236 |
| | > 25 µm/mL | 1 | 1 | 3 | 3 |
| | > 50 µm/mL | 0 | 0 | 0 | 0 |

| | | | | | |
|---|---|---|---|---|---|
| BQL states for below quantification limit ND states for not detected NP states for not performed Complies means clear solution, essentially free from visible particles | | | | | |

The particulate matter was measured according to USP-NF 42-37 <789> *Particulate matter in ophthalmic solutions* wherein it defines the acceptable limits for particulate matter as: 50 for > 10 µm/mL, 5 for > 25 µm/mL and 2 for > 50 µm/mL.

### Example 7 - Composition 9 containing Levofloxacin hemihydrate and Ketorolac tromethamine without preservative.

**TABLE 13: Composition 9 of the present invention.**

| | **Composition 9** | |
|---|---|---|
| **Ingredient** | **Concentration (mg/mL)** | **Function** |
| Levofloxacin hemihydrate | 5.12 (5.00 on the anhydrous basis) | Active ingredient, anti-infective |
| Ketorolac tromethamine | 5.00 | Active ingredient, NSAID |
| Sodium chloride | q.s. 280-420 mOsm/kg osmolality | Tonicity agent |
| Disodium EDTA | 1.00 | Chelating agent |
| Sodium hydroxide or Hydrochloric acid | q.s. pH 8.0 | pH adjusting agent |
| Water for injection | q.s. 1 mL | Vehicle |

Composition 9 of Example 7 of the present invention was prepared according to the same manufacturing process of Composition 3 without the addition of any preservative and the intermediate pH value was adjusted to approximately 9.0 and the pH of the final product was adjusted to 8.0 with the addition of either hydrochloric acid or sodium hydroxide.

The bottles of Composition 9 were exposed to long-term (25±2°C, 60±5% relative humidity) stability studies. The stability results of Composition 9 over 1 month (see Table 14) were promising giving potential to a preservative free composition in combination with a suitable container, either single-dose or multi-dose bottle designed specifically to store preservative-free ophthalmic solutions.

**TABLE 14: Stability results of Composition 9 at long-term conditions (25±2°C, 60±5% relative humidity)**

| Appearance | Complies |
|---|---|
| Levofloxacin Assay (anhydrous basis) (%) | 101.9 |
| Ketorolac assay (%) | 101.1 |
| Total related substances of Levofloxacin (%) | <0.05 |
| Total related substances of Ketorolac (%) | ND |
| Osmolality | 328 |
| Final pH | 8.0 |

| | |
|---|---|
| ND states for not detected Complies means clear solution, essentially free from visible particles | |

With the present invention it was observed that independently of the use of solubilizers or other excipients, the combination of ketorolac tromethamine and levofloxacin will only remain stable and not precipitate in the case the pH value of the final solution is between 7.5-8.5. Further, the intermediate pH value adjustment during the manufacturing process to values between 8.5 and 9.5, adds robustness to the overall stability of the final product. In addition, the two active ingredients seem to be compatible at a pH value of 7.5-8.5 of the final product, only when they are combined in specific amounts up to approximately 9.0 mg/mL.

While the present invention has been described with respect to the particular embodiments, it will be apparent to those skilled in the art that various changes and modifications may be made in the invention without departing from the spirit and scope thereof, as defined in the appended claims.

## Claims

1. An ophthalmic composition in the form of an aqueous solution comprising a therapeutically effective amount of antibiotic levofloxacin or a pharmaceutically acceptable salt thereof, as a first active ingredient, and a therapeutically effective amount of non-steroidal anti-inflammatory and analgesic ketorolac or a pharmaceutically acceptable salt thereof, as a second active ingredient, wherein said first active ingredient is from 0.4 to 0.9%w/v and said second active ingredient is from 0.4 to 0.9%w/v, wherein the weight/volume percentages are expressed as g/100mL units, and wherein said composition has a pH value ranging from 7.7 to 8.5.

2. The ophthalmic composition according to claim 1, wherein said pH value ranges from 7.9 to 8.3 and more preferably is 8.0.

3. The ophthalmic composition according to claims 1-2, wherein said composition comprises levofloxacin in the hemihydrate form and ketorolac tromethamine.

4. The ophthalmic composition according to any one of claims 1-3, wherein said composition comprises 0.5% w/v levofloxacin and 0.5% w/v ketorolac tromethamine.

5. The ophthalmic composition according to any preceding claim, wherein said composition further comprises at least one of the ophthalmically acceptable excipients selected from preservatives, pH adjusting agents, solubilizers, chelating agents and tonicity agents.

6. The ophthalmic composition according to claim 5, wherein independently from one another, the tonicity agent is sodium chloride, the chelating agent is disodium EDTA, the preservative is benzalkonium chloride, the pH adjusting agent is sodium hydroxide or hydrochloric acid.

7. The ophthalmic composition according to claim 5 or 6, wherein said composition further comprises polysorbate as solubilizer.

8. The ophthalmic composition according to any preceding claim, wherein said composition does not comprise any preservative.

9. A process for the preparation of a stable ophthalmic composition in the form of an aqueous solution comprising a therapeutically effective amount of antibiotic levofloxacin or a pharmaceutically acceptable salt thereof, as a first active ingredient, and a therapeutically effective amount of non-steroidal anti-inflammatory and analgesic ketorolac or a pharmaceutically acceptable salt thereof, as a second active ingredient, wherein said first active ingredient is from 0.4 to 0.9% w/v and said second active ingredient is from 0.4 to 0.9% w/v, wherein the weight/volume percentages are expressed as g/100mL units, wherein said process comprises the following steps:
(a) dissolving in water for injection at least one of the excipients selected from tonicity agents, chelating agents and/or preservatives;
(b) adding the total amount of ketorolac or salt thereof, to the resulting solution of step (a) and stirring for appropriate time until completely dissolved and adjusting the pH to a value between 8.5 to 9.5 with suitable hydrochloric acid or sodium hydroxide solution;
(c) adding the total amount of levofloxacin or salt thereof to the solution of step (b) and stirring until completely dissolved;
(d) adjusting the pH of the final solution to a pH value between 7.5 to 8.5, preferably from 7.7 to 8.5, more preferably from 7.9 to 8.3 and yet more preferably 8.0, with suitable hydrochloric acid or sodium hydroxide solution.

10. A process for the preparation of a stable ophthalmic composition in the form of an aqueous solution comprising a therapeutically effective amount of antibiotic levofloxacin or a pharmaceutically acceptable salt thereof, as a first active ingredient, and a therapeutically effective amount of non-steroidal anti-inflammatory and analgesic ketorolac or a pharmaceutically acceptable salt thereof, as a second active ingredient, wherein said first active ingredient is from 0.4 to 0.9% w/v and said second active ingredient is from 0.4 to 0.9% w/v, wherein the weight/volume percentages are expressed as g/100mL units, wherein said process comprises the following steps:
(a) dissolving in water for injection at least one of the excipients selected from tonicity agents, chelating agents and/or preservatives;
(b) adding the total amount of levofloxacin or salt thereof, to the resulting solution of step (a) and stirring for appropriate time until completely dissolved and adjusting the pH to a value between 8.5 to 9.5 with suitable hydrochloric acid or sodium hydroxide solution;
(c) adding the total amount of ketorolac or salt thereof to the solution of step (b) and stirring until completely dissolved;
(d) adjusting the pH of the final solution to a pH value between 7.5 to 8.5, preferably from 7.7 to 8.5, more preferably from 7.9 to 8.3 and yet more preferably 8.0, with suitable hydrochloric acid or sodium hydroxide solution.

11. The process for the preparation of a composition according to claim 9 or 10, further comprising a step of filtering the solution through a sterilizing filter, which preferably has a nominal porosity of 0.2 µm.

12. The process according to claim 11, wherein the filtered solution is filled in single-dose or multi-dose bottles provided with a dropper and a screw cap.

13. The process for the preparation of a composition according to claim 9 or 10, wherein in step (a) no preservative is added.

14. The process for the preparation of a composition according to claim 9 or 10, wherein in step (a) polysorbate is added as solubilizer.

15. An ophthalmic composition according to claims 1-8 for the prevention or therapeutic treatment of inflammation and/or infections of the eye, in particular conjunctivitis optionally associated with infections of bacterial or viral origin.

## Patentansprüche

1. Ophthalmische Zusammensetzung in Form einer wässrigen Lösung, umfassend eine therapeutisch wirksame Menge von antibiotischem Levofloxacin oder einem pharmazeutisch annehmbaren Salz davon als einen ersten Wirkstoff und eine therapeutisch wirksame Menge von nichtsteroidalem entzündungshemmendem und analgetischem Ketorolac oder einem pharmazeutisch annehmbaren Salz davon als einen zweiten Wirkstoff, wobei der erste Wirkstoff 0,4 bis 0,9 % Gew./Vol. beträgt und der zweite Wirkstoff 0,4 bis 0,9 % Gew./Vol. beträgt, wobei die Gewichts-/Volumenprozentsätze als g/100 ml-Einheiten ausgedrückt sind und wobei die Zusammensetzung einen pH-Wert im Bereich von 7,7 bis 8,5 aufweist.

2. Ophthalmische Zusammensetzung nach Anspruch 1, wobei der pH-Wert im Bereich von 7,9 bis 8,3 liegt und bevorzugter 8,0 beträgt.

3. Ophthalmische Zusammensetzung nach den Ansprüchen 1-2, wobei die Zusammensetzung Levofloxacin in der Hemihydratform und Ketorolactromethamin umfasst.

4. Ophthalmische Zusammensetzung nach einem der Ansprüche 1-3, wobei die Zusammensetzung 0,5 % Gew./Vol. Levofloxacin und 0,5 % Gew./Vol. Ketorolactromethamin umfasst.

5. Ophthalmische Zusammensetzung nach einem der vorhergehenden Ansprüche, wobei die Zusammensetzung ferner mindestens einen der ophthalmisch annehmbaren Hilfsstoffe umfasst, ausgewählt aus Konservierungsmitteln, pH-Einstellmitteln, Lösungsvermittlern, Chelatbildnern und Tonizitätsmitteln.

6. Ophthalmische Zusammensetzung nach Anspruch 5, wobei unabhängig voneinander das Tonizitätsmittel Natriumchlorid ist, der Chelatbildner Dinatrium-EDTA ist, das Konservierungsmittel Benzalkoniumchlorid ist, das pH-Einstellmittel Natriumhydroxid oder Salzsäure ist.

7. Ophthalmische Zusammensetzung nach Anspruch 5 oder 6, wobei die Zusammensetzung ferner Polysorbat als Lösungsvermittler umfasst.

8. Ophthalmische Zusammensetzung nach einem der vorhergehenden Ansprüche, wobei die Zusammensetzung kein Konservierungsmittel umfasst.

9. Verfahren zur Herstellung einer stabilen ophthalmischen Zusammensetzung in Form einer wässrigen Lösung, umfassend eine therapeutisch wirksame Menge von antibiotischem Levofloxacin oder einem pharmazeutisch annehmbaren Salz davon als einen ersten Wirkstoff und eine therapeutisch wirksame Menge von nichtsteroidalem entzündungshemmendem und analgetischem Ketorolac oder einem pharmazeutisch annehmbaren Salz davon als einen zweiten Wirkstoff, wobei der erste Wirkstoff 0,4 bis 0,9 % Gew./Vol. beträgt und der zweite Wirkstoff 0,4 bis 0,9 % Gew./Vol. beträgt, wobei die Gewichts-/Volumenprozentsätze als g/100 ml-Einheiten ausgedrückt sind, wobei das Verfahren die folgenden Schritte umfasst:
(a) Auflösen in Wasser zur Injektion von mindestens einem der Hilfsstoffe, ausgewählt aus Tonizitätsmitteln, Chelatbildnern und/oder Konservierungsmitteln;
(b) Zugeben der Gesamtmenge von Ketorolac oder eines Salzes davon zu der resultierenden Lösung von Schritt (a) und Rühren für eine geeignete Zeit bis zur vollständigen Auflösung und Einstellen des pH-Werts auf einen Wert zwischen 8,5 bis 9,5 mit geeigneter Salzsäure- oder Natriumhydroxidlösung;
(c) Zugeben der Gesamtmenge von Levofloxacin oder eines Salzes davon zu der Lösung von Schritt (b) und Rühren bis zur vollständigen Auflösung;
(d) Einstellen des pH-Werts der endgültigen Lösung auf einen pH-Wert zwischen 7,5 bis 8,5, vorzugsweise von 7,7 bis 8,5, bevorzugter von 7,9 bis 8,3 und noch bevorzugter 8,0, mit geeigneter Salzsäure- oder Natriumhydroxidlösung.

10. Verfahren zur Herstellung einer stabilen ophthalmischen Zusammensetzung in Form einer wässrigen Lösung, umfassend eine therapeutisch wirksame Menge von antibiotischem Levofloxacin oder einem pharmazeutisch annehmbaren Salz davon als einen ersten Wirkstoff und eine therapeutisch wirksame Menge von nichtsteroidalem entzündungshemmendem und analgetischem Ketorolac oder einem pharmazeutisch annehmbaren Salz davon als einen zweiten Wirkstoff, wobei der erste Wirkstoff 0,4 bis 0,9 % Gew./Vol. beträgt und der zweite Wirkstoff 0,4 bis 0,9 % Gew./Vol. beträgt, wobei die Gewichts-/Volumenprozentsätze als g/100 ml-Einheiten ausgedrückt sind, wobei das Verfahren die folgenden Schritte umfasst:
(a) Auflösen in Wasser zur Injektion von mindestens einem der Hilfsstoffe, ausgewählt aus Tonizitätsmitteln, Chelatbildnern und/oder Konservierungsmitteln;
(b) Zugeben der Gesamtmenge von Levofloxacin oder eines Salzes davon zu der resultierenden Lösung von Schritt (a) und Rühren für eine geeignete Zeit bis zur vollständigen Auflösung und Einstellen des pH-Werts auf einen Wert zwischen 8,5 bis 9,5 mit geeigneter Salzsäure- oder Natriumhydroxidlösung;
(c) Zugeben der Gesamtmenge von Ketorolac oder eines Salzes davon zu der Lösung von Schritt (b) und Rühren bis zur vollständigen Auflösung;
(d) Einstellen des pH-Werts der endgültigen Lösung auf einen pH-Wert zwischen 7,5 bis 8,5, vorzugsweise von 7,7 bis 8,5, bevorzugter von 7,9 bis 8,3 und noch bevorzugter 8,0, mit geeigneter Salzsäure- oder Natriumhydroxidlösung.

11. Verfahren zur Herstellung einer Zusammensetzung nach Anspruch 9 oder 10, ferner umfassend einen Schritt des Filtrierens der Lösung durch einen Sterilisationsfilter, der vorzugsweise eine nominelle Porosität von 0,2 µm aufweist.

12. Verfahren nach Anspruch 11, wobei die filtrierte Lösung in Einzeldosis- oder Multidosisflaschen gefüllt wird, die mit einem Tropfer und einer Schraubkappe versehen sind.

13. Verfahren zur Herstellung einer Zusammensetzung nach Anspruch 9 oder 10, wobei in Schritt (a) kein Konservierungsmittel zugegeben wird.

14. Verfahren zur Herstellung einer Zusammensetzung nach Anspruch 9 oder 10, wobei in Schritt (a) Polysorbat als Lösungsvermittler zugegeben wird.

15. Ophthalmische Zusammensetzung nach den Ansprüchen 1-8 zur Prävention oder therapeutischen Behandlung von Entzündungen und/oder Infektionen des Auges, insbesondere Konjunktivitis, gegebenenfalls assoziiert mit Infektionen bakteriellen oder viralen Ursprungs.

## Revendications

1. Composition ophtalmique sous la forme d'une solution aqueuse comprenant une quantité thérapeutiquement efficace d'antibiotique lévofloxacine ou d'un sel pharmaceutiquement acceptable de celui-ci, en tant que premier principe actif, et une quantité thérapeutiquement efficace d'anti-inflammatoire non stéroïdien et d'analgésique kétorolac ou d'un sel pharmaceutiquement acceptable de celui-ci, en tant que second principe actif, dans laquelle ledit premier principe actif est présent de 0,4 à 0,9 % m/v et ledit second principe actif est présent de 0,4 à 0,9 % m/v, dans laquelle les pourcentages en masse/volume sont exprimés en g/100 ml, et dans laquelle ladite composition a une valeur de pH entre 7,7 et 8,5.

2. Composition ophtalmique selon la revendication 1, dans laquelle ladite valeur de pH s'étend de 7,9 à 8,3 et plus préférablement est de 8,0.

3. Composition ophtalmique selon les revendications 1 à 2, dans laquelle ladite composition comprend de la lévofloxacine sous forme hémihydratée et du kétorolac trométhamine.

4. Composition ophtalmique selon l'une quelconque des revendications 1 à 3, dans laquelle ladite composition comprend 0,5 % m/v de lévofloxacine et 0,5 % m/v de kétorolac trométhamine.

5. Composition ophtalmique selon l'une quelconque des revendications précédentes, dans laquelle ladite composition comprend en outre au moins un des excipients ophtalmiquement acceptables choisis parmi les conservateurs, les agents régulateurs de pH, les solubilisants, les agents chélatants et les agents de tonicité.

6. Composition ophtalmique selon la revendication 5, dans laquelle, indépendamment les uns des autres, l'agent de tonicité est le chlorure de sodium, l'agent chélatant est l'EDTA disodique, le conservateur est le chlorure de benzalkonium, l'agent régulateur de pH est l'hydroxyde de sodium ou l'acide chlorhydrique.

7. Composition ophtalmique selon la revendication 5 ou 6, dans laquelle ladite composition comprend en outre du polysorbate en tant que solubilisant.

8. Composition ophtalmique selon l'une quelconque des revendications précédentes, dans laquelle ladite composition ne comprend aucun conservateur.

9. Procédé de préparation d'une composition ophtalmique stable sous la forme d'une solution aqueuse comprenant une quantité thérapeutiquement efficace d'antibiotique lévofloxacine ou d'un sel pharmaceutiquement acceptable de celui-ci, en tant que premier principe actif, et une quantité thérapeutiquement efficace d'anti-inflammatoire non stéroïdien et d'analgésique kétorolac ou d'un sel pharmaceutiquement acceptable de celui-ci, en tant que second principe actif, dans lequel ledit premier principe actif est présent de 0,4 à 0,9 % m/v et ledit second principe actif est présent de 0,4 à 0,9 % m/v, dans lequel les pourcentages en masse/volume sont exprimés en g/100 ml, dans lequel ledit procédé comprend les étapes suivantes :
(a) dissolution dans de l'eau pour préparation injectable d'au moins un des excipients choisis parmi les agents de tonicité, les agents chélatants et/ou les conservateurs ;
(b) ajout de la quantité totale de kétorolac ou du sel de celui-ci, à la solution obtenue de l'étape (a) et mélange pendant la durée appropriée jusqu'à dissolution complète et ajustement du pH à une valeur entre 8,5 et 9,5 avec une solution d'acide chlorhydrique ou d'hydroxyde de sodium appropriée ;
(c) ajout de la quantité totale de lévofloxacine ou du sel de celle-ci à la solution de l'étape (b) et mélange jusqu'à dissolution complète ;
(d) ajustement du pH de la solution finale à une valeur de pH entre 7,5 et 8,5, de préférence entre 7,7 et 8,5, plus préférablement entre 7,9 et 8,3 et encore plus préférablement de 8,0, avec une solution d'acide chlorhydrique ou d'hydroxyde de sodium appropriée.

10. Procédé de préparation d'une composition ophtalmique stable sous la forme d'une solution aqueuse comprenant une quantité thérapeutiquement efficace d'antibiotique lévofloxacine ou d'un sel pharmaceutiquement acceptable de celui-ci, en tant que premier principe actif, et une quantité thérapeutiquement efficace d'anti-inflammatoire non stéroïdien et d'analgésique kétorolac ou d'un sel pharmaceutiquement acceptable de celui-ci, en tant que second principe actif, dans lequel ledit premier principe actif est présent de 0,4 à 0,9 % m/v et ledit second principe actif est présent de 0,4 à 0,9 % m/v, dans lequel les pourcentages en masse/volume sont exprimés en g/100 ml, dans lequel ledit procédé comprend les étapes suivantes :
(a) dissolution dans de l'eau pour préparation injectable d'au moins un des excipients choisis parmi les agents de tonicité, les agents chélatants et/ou les conservateurs ;
(b) ajout de la quantité totale de lévofloxacine ou du sel de celle-ci, à la solution obtenue de l'étape (a) et mélange pendant la durée appropriée jusqu'à dissolution complète et ajustement du pH à une valeur entre 8,5 et 9,5 avec une solution d'acide chlorhydrique ou d'hydroxyde de sodium appropriée ;
(c) ajout de la quantité totale de kétorolac ou du sel de celui-ci à la solution de l'étape (b) et mélange jusqu'à dissolution complète ;
(d) ajustement du pH de la solution finale à une valeur de pH entre 7,5 et 8,5, de préférence entre 7,7 et 8,5, plus préférablement entre 7,9 et 8,3 et encore plus préférablement de 8,0, avec une solution d'acide chlorhydrique ou d'hydroxyde de sodium appropriée.

11. Procédé de préparation d'une composition selon la revendication 9 ou 10, comprenant en outre une étape de filtration de la solution à travers un filtre stérilisant, ayant de préférence une porosité nominale de 0,2 µm.

12. Procédé selon la revendication 11, dans lequel la solution filtrée est versée dans des flacons unidoses ou multidoses munis d'un compte-gouttes et d'un capuchon à visser.

13. Procédé de préparation d'une composition selon la revendication 9 ou 10, dans lequel dans l'étape (a) aucun conservateur n'est ajouté.

14. Procédé de préparation d'une composition selon la revendication 9 ou 10, dans lequel dans l'étape (a) du polysorbate est ajouté en tant que solubilisant.

15. Composition ophtalmique selon les revendications 1 à 8 pour la prévention ou le traitement thérapeutique d'inflammation et/ou d'infections de l'œil, en particulier de la conjonctivite facultativement associée à des infections d'origine bactérienne ou virale.
